(19) Europäisches Patentamt
European Patent Office
Office européen des brevets

(11) Veröffentlichungsnummer: **0 087 615**
**A1**

# (12) EUROPÄISCHE PATENTANMELDUNG

(21) Anmeldenummer: **83101128.3**

(22) Anmeldetag: **07.02.83**

(51) Int. Cl.³: **C 07 D 231/16**
C 07 F 9/65, A 01 N 57/16
//C07D261/08

(30) Priorität: **16.02.82 DE 3205455**
**16.02.82 DE 3205456**

(43) Veröffentlichungstag der Anmeldung:
**07.09.83 Patentblatt 83/36**

(84) Benannte Vertragsstaaten:
**AT BE CH DE FR GB IT LI NL**

(71) Anmelder: **BASF Aktiengesellschaft**
**Carl-Bosch-Strasse 38**
**D-6700 Ludwigshafen(DE)**

(72) Erfinder: **Rieber, Norbert, Dr.**
**Liebfrauenstrasse 1C**
**D-6800 Mannheim(DE)**

(72) Erfinder: **Boehm, Heinrich, Dr.**
**Keplerweg 2**
**D-6708 Neuhofen(DE)**

(72) Erfinder: **Adolphi, Heinrich, Dr.**
**Kalmitweg 11**
**D-6703 Limburgerhof(DE)**

(54) **Halogenierte 1-Hydroxipyrazole und Verfahren zu ihrer Herstellung.**

(57) 1-Pryazolyl-phosphorsäureester der Formel

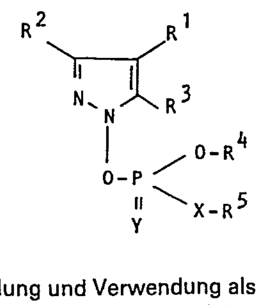

deren Herstellung und Verwendung als Pflanzenschutzmittel sowie deren unmittelbare Vorprodukte, nämlich halogenierte 1-Hydroxipyrazole der Formel

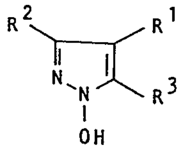

wobei $R^1$ bis $R^5$, X und Y die in Ansprüchen und Beschreibung angegebene Bedeutung haben.

EP 0 087 615 A1

# Halogenierte 1-Hydroxipyrazole und Verfahren zu ihrer Herstellung

Die Erfindung betrifft halogenierte 1-Hydroxipyrazole der allgemeinen Formel (I)

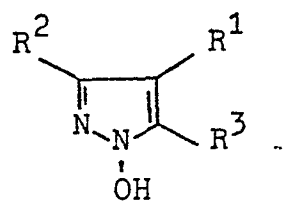

(I),

in der $R^1$, $R^2$ und $R^3$ unabhängig voneinander Wasserstoff, Chlor, Brom oder Iod bedeuten, mit der Maßgabe, daß $R^1$, $R^2$ und $R^3$ nicht gleichzeitig für Wasserstoff stehen, die wertvolle Zwischenprodukte darstellen und aus diesen erhältliche neue 1-Pyrazolyl-phosphorsäureester der allgemeinen Formel (II), Verfahren zu deren Herstellung, Schädlingsbekämpfungsmittel, die diese Verbindungen als Wirkstoffe enthalten, sowie Verfahren zur Bekämpfung von Schädlingen mit diesen Wirkstoffen bzw. Mitteln.

Die erfindungsgemäßen Verbindungen der Formel I können durch Halogenierung von 1-Hydroxipyrazol nach an sich bekannten Methoden erhalten werden (Houben-Weyl, Methoden der org. Chemie, Bd. V/3, 725ff, Georg Thieme-Verlag, Stuttgart, 4. Auflage, 1962; Bd. V/4, 38ff, 530ff, Georg Thieme-Verlag, Stuttgart, 4. Auflage, 1960).

Je nach Menge des eingesetzten Halogenierungsmittels entstehen dabei die entsprechenden Mono-, Di- oder Trihalogen-Derivate. Die Mehrfach-Halogenierung kann stufenweise oder in einem Schritt durchgeführt werden. Durch stufenweise Halogenierung lassen sich auch Derivate mit verschiedenen Halogenresten synthetisieren.

Mu/IG

Im einzelnen wird die Halogenierung meist in Anwesenheit von inerten Lösungsmitteln, z.B. halogenierter Kohlenwasserstoffe, mit oder ohne Zusatz von Hilfsbasen, z.B. Alkalicarbonate, durch Zugabe des Halogenierungsmittels, z.B. der Halogene, von N-Halogenverbindungen und Hypohalogeniten, bei Temperaturen zwischen -60 und +100°C, vorzugsweise bei -20 bis +80°C, vorgenommen. Die Komponenten können dabei im molaren Über- oder Unterschuß eingesetzt werden. Die Menge an Hilfsbase beträgt zweckmäßigerweise 1 bis 25 Mol pro Mol 1-Hydroxipyrazol.

Die Isolierung der neuen Verbindungen der Formel I erfolgt nach üblichen Methoden, z.B. durch Abfiltrieren, Extraktion aus der Reaktionslösung oder Einengen des Reaktionsgemisches und/oder fraktionierte Kristallisation.

Das als Ausgangsstoff eingesetzte 1-Hydroxipyrazol erhält man im Gemisch mit Isoxazolen der Formel

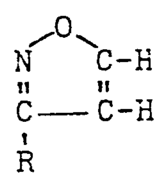

worin R ein Wasserstoffatom, einen aliphatischen, cycloaliphatischen, araliphatischen, aromatischen Rest bedeutet,
durch Erhitzen von Isoxazolin-azoxiverbindungen der Formel Ia

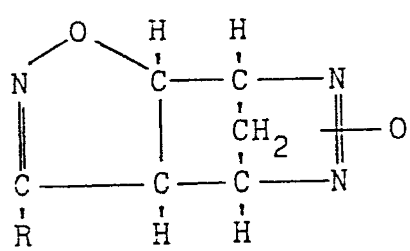

(Ia ),

worin R die vorgenannte Bedeutung hat, auf eine Temperatur zwischen 140 bis 600°C.

Die Umsetzung kann für den Fall der Verwendung von 2,3-Azoxi-6-oxa-7-aza-tricyclo-[5.2.1.0$^{5.9}$]-deca-2,7-dien durch die folgenden Formeln wiedergegeben werden:

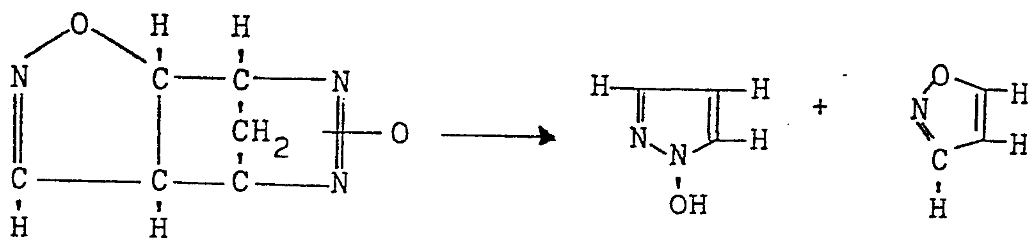

Bevorzugte Ausgangsstoffe Ia sind solche, in deren Formeln R ein Wasserstoffatom, einen Alkylrest mit 1 bis 18, insbesondere 1 bis 6 Kohlenstoffatomen, einen Cycloalkylrest mit 5 bis 8 Kohlenstoffatomen, einen Aralkylrest mit 7 bis 12 Kohlenstoffatomen, einen unsubstituierten oder durch Bromatome, Fluoratome, Chloratome, Alkylgruppen und/oder Alkoxygruppen mit 1 bis 4 Kohlenstoffatomen substituierten Phenylrest bedeutet. Die vorgenannten Reste können noch durch unter den Reaktionsbedingungen inerte Gruppen und/oder Atome, z.B. Alkylgruppen und Alkoxygruppen mit jeweils 1 bis 4 Kohlenstoffatomen, Phenylreste substituierende Bromatome, Fluoratome, Chloratome, substituiert sein.

Die Ausgangsstoffe Ia können leicht durch Umsetzung von 2,3,7-Triaza-6-oxa-tricyclo-[5.2.1.0$^{5.9}$]-deca-2,7-dienen der Formel Ib

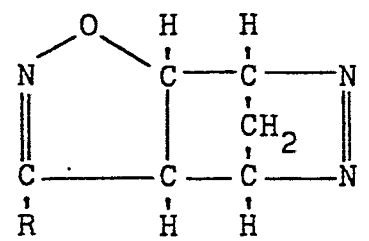

(Ib),

worin R die vorgenannte Bedeutung hat, mit einem organischen Peroxid bei einer Temperatur von -10 bis +130°C, vorzugsweise 20 bis 100°C, insbesondere 50 bis 90°C, drucklos oder unter Druck, kontinuierlich oder diskontinuierlich, in Abwesenheit oder Anwesenheit von organischen Lösungsmitteln wie Halogenkohlenwasserstoffe und Ether, erhalten werden. Das Peroxid kann in stöchiometrischer Menge oder im Überschuß, bevorzugt in einem Verhältnis von 1 bis 10, insbesondere 1 bis 1,5 Mol Peroxid je Mol Ausgangsstoff umgesetzt werden.

Die Ausgangsstoffe Ib können leicht erhalten werden, indem man in einem ersten Schritt Nitriloxide der Formel Ic

$$R^1CNO \quad (Ic),$$

worin $R^1$ die vorgenannte allgemeine und bevorzugte Bedeutung hat, mit N,N'-Dicarbalkoxy-2,3-diaza-bicyclo-[2.2.1]-hept-2-enen der Formel Id

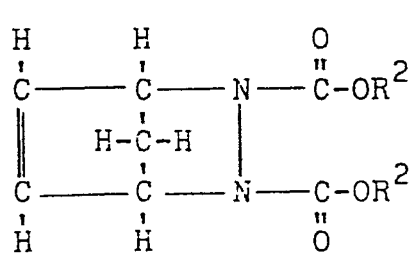

(Id),

worin die einzelnen Reste $R^2$ gleich oder verschieden sein können und jeweils ein Wasserstoffatom oder einen aliphatischen Rest bedeuten umsetzt, und dann in einem 2. Schritt die so erhaltenen N,N'-Dicarbalkoxy-isoxazolino-Verbindungen der Formel Ie

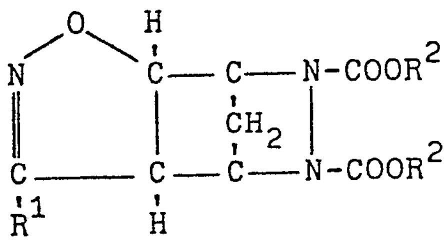

(Ie),

worin $R^1$ die vorgenannte Bedeutung besitzt, nach üblicher Methode verseift, decarboxyliert und zu den Ausgangsstoffen III oxidiert.

Die Ausgangsstoffe Ic sind leicht durch die in Houben-Weyl, Methoden der Organischen Chemie, Band 10/3, Seiten 841 bis 853, beschriebenen Arbeitsweise, z.B. durch Dehydrierung von Aldoximen oder aus Hydroxamsäurederivaten oder Nitrolsäuren zugänglich. Die Ausgangsstoffe Id erhält man z.B. durch Umsetzung von Cyclopentadien mit Azodicarbonsäuredimethylestern nach den in Ann. 443, 242 bis 262 (1925) beschriebenen Verfahren. Der Sauerstoff der Azoxigruppe kann sowohl am einen wie am anderen Stickstoffatom der Azogruppierung gebunden sein. Daher können als Ausgangsstoffe Ia die reinen Isomere

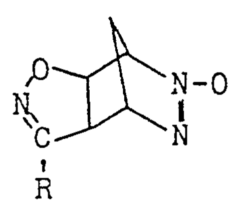

und

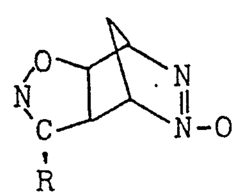

r zweckmäßig das Isomerengemisch, wie es bei der Herllung anfällt, verwendet werden.

So kommen beispielsweise die folgenden Ausgangsstoffe Ia infrage: 2,3-Azoxi-6-oxa-7-aza-tricyclo-[5.2.1.$0^{5.9}$]-deca-2,7-dien und seine in 8-Stellung durch die Methyl-, Ethyl-, Propyl-, Isopropyl-, Butyl-, Isobutyl-, sek.-Butyl, tert.-Butyl-, Cyclohexyl-, Cyclopentyl-, Benzyl-, Phenyl-, 2'-Chlorphenyl-, 3'-Chlorphenyl-, 4'-Chlorphenyl-, 2'-Methylphenyl-, 3'-Methylphenyl-, 4'-Methylphenyl-, 2'-Methoxyphenyl-, 3'-Methoxyphenyl-, 4'-Methoxyphenyl-, 2'-Ethylphenyl-, 3'-Ethylphenyl-, 4'-Ethylphenyl-, 2'-Ethoxyphenyl-, 3'-Ethoxyphenyl-, 4'-Ethoxyphenylgruppe substituierten Homologen.

Die Umsetzung wird bei einer Temperatur von 140 bis 600°C, vorzugsweise 150 bis 500°C, im Falle von Ausgangsstoffen Ia mit R in der Bedeutung Wasserstoff, aliphatischem, cycloaliphatischem, araliphatischem Rest zweckmäßig bei einer Temperatur von 200 bis 600, vorzugsweise 250 bis 500, insbesondere 300 bis 450°C, im Falle von Ausgangsstoffen Ia mit R in der Bedeutung aromatischem Rest von 140 bis 200°C, vorzugsweise 150 bis 190°C, insbesondere 160 bis 180°C, drucklos, mit Unterdruck oder unter Druck, diskontinuierlich oder kontinuierlich durchgeführt. Man kann unter den Reaktionsbedingungen inerte, organische Lösungsmittel verwenden oder schon aus wirtschaftlichen Gründen zweckmäßigerweise in Abwesenheit organischer Lösungsmittel umsetzen.

Die Reaktion kann wie folgt durchgeführt werden: Der Ausgangsstoff Ia wird während einer Sekunde bis 21 Stunden bei der Reaktionstemperatur gehalten. Dann werden das 1-Hydroxipyrazol und das Isoxazol aus dem Reaktionsgemisch in üblicher Weise, z.B. durch fraktionierte Destillation bzw. Kondensation, Extraktion oder Kristallisation, abgetrennt.

1-Hydroxipyrazol kann beispielsweise auf folgendem Wege erhalten werden:

97 Gew.Teile 8-Methyl-2,3-azoxi-6-oxa-7-aza-tricyclo-[$5.2.1.0^{5.9}$]-decy-2,7-dien werden innerhalb von 2 Stunden bei 455°C und 5 mbar durch einen mit 300 Teilen Quarzringe gefüllten Rohrreaktor geführt. Das Reaktionsgemisch wird in 2 Vorlagen fraktioniert kondensiert. In der ersten auf 5°C gekühlten Vorlage erhält man 47 Gew.Teile (97 % der Theorie) 1-Hydroxipyrazol vom Fp 75°C (aus Ligroin). Die zweite auf -70°C gekühlte Vorlage enthält 46 Gew.Teile (95 % der Theorie) 3-Methyl-isoxazol vom Kp 117°C.

Die folgenden Beispiele A bis D erläutern die Herstellung erfindungsgemäßer Verbindungen des Typs I.

Beispiel

A. Zu 20 Gew.-Teilen 1-Hydroxipyrazol und 50 Gew.-Teilen $Na_2CO_3$ in 700 Gew.-Teilen Methylenchlorid gibt man eine Lösung von 42 Gew.-Teilen Brom in 300 Gew.-Teilen Methylenchlorid unter Rühren bei 0°C. Nach der Zugabe wird noch 1 Stunde nachgerührt. Anschließend filtriert man ab, engt das Filtrat im Rotationsverdampfer bei 20 bis 40°C/20 mbar ein und kristallisiert den so erhaltenen Rückstand aus Ligroin um.

Austrag: 39 Gew.-Teile (91 % der Theorie) 1-Hydroxi-4--brompyrazol: Fp. 136°C.

B. Zu 4 Gew.-Teilen 1-Hydroxipyrazol und 10 Gew.-Teilen $Na_2CO_3$ in 1000 Gew.-Teilen Chloroform fügt man bei Rückflußtemperatur unter Rühren eine Lösung von 36 Teilen Iod in 1500 Teilen Chloroform. Nach der Zugabe rührt man noch 24 Stunden unter Rückfluß nach.

Anschließend wird das Festprodukt abgesaugt, in 200 Gew.-Teilen Wasser suspendiert und mit 10 proz. Salzsäure auf pH = 4 angesäuert. Das Festprodukt wird abgesaugt, in Methylenchlorid gelöst, die Lösung mit Magnesiumsulfat getrocknet, abfiltriert und im Rotationsverdampfer bei 20 bis 40°C und 20 mbar eingeengt. Der Rückstand wird in Toluol umkristallisiert.

Austrag: 13,5 Gew.-Teile (61 % der Theorie) 1-Hydroxi-3,4,5-triiodpyrazol; Fp. 153°C (Zers.).

C. Zu 3 Gew.-Teilen 1-Hydroxipyrazol und 1 Gew.-Teil Ethylendiamin (als 60 proz. wäßrige Lösung) in 10 Gew.-Teilen Ethanol fügt man unter Rühren bei 20°C 9 Gew.-Teile Iod in 50 Gew.-Teilen gesättigter wäßriger Kaliumiodidlösung. Nach der Zugabe wird noch 2 Stunden nachgerührt. Das Reaktionsgemisch wird anschließend in eine verdünnte wäßrige Natriumthiosulfatlösung gegossen. Das Festprodukt wird abgesaugt, in Methylenchlorid gelöst, die Lösung mit Magnesiumsulfat getrocknet und im Rotationsverdampfer bei 20 bis 40°C und 20 mbar eingeengt. Der Rückstand wird aus Ligroin umkristallisiert.

Austrag: 6 Gew.-Teile (80 % der Theorie) 1-Hydroxi-4-iodpyrazol; Fp. 126°C.

D. Zu 30 Gew.-Teilen 1-Hydroxi-4-chlorpyrazol und 0,5 Gew.-Teilen Eisenpulver in 300 Gew.-Teilen 1,2-Dichlorethan fügt man unter Rühren bei 60°C 81 Gew.-Teile Brom. Danach rührt man noch 2 Stunden bei 60°C nach. Anschließend wird das Festprodukt abgesaugt und aus Ligroin umkristallisiert.

Austrag: 55 Gew.-Teile (78 % der Theorie) 1-Hydroxi-3,5-dibrom-4-chlorpyrazol; Fp. 160°C.

Entsprechend den Beispielen A, B, C oder D können folgende Verbindungen der Formel I erhalten werden:

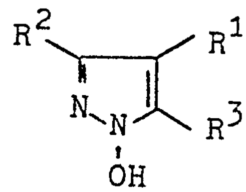

| $R^1$ | $R^2$ | $R^3$ | Fp. [°C] |
|---|---|---|---|
| Cl | H | H | 122 |
| Br | Br | H | 162 |
| Br | Cl | H | |
| Br | I | H | |
| Cl | Br | H | |
| Cl | I | H | |
| Cl | Cl | H | 138 |
| I | Br | H | |
| I | Cl | H | |
| Cl | Cl | Cl | 172 |
| Br | Cl | Cl | |
| Cl | I | I | |
| Br | Br | Br | 169 |
| Br | I | I | |
| I | Br | Br | |
| I | Cl | Cl | |

Durch Umsetzung der halogenierten 1-Hydroxipyrazole der Formel I mit Phosphorsäureesterhalogeniden IIa erhält man 1-Pyrazolylphosphorsäureester (II), die sich zur Bekämpfung von tierischen Schädlingen eignen und sich durch eine besonders hohe spezifische Wirksamkeit auszeichnen.

1-Pyrazolyl-phosphorsäureester sind bisher in der Literatur nicht beschrieben. Bekannt sind lediglich 4- bzw. 5-Pyrazolyl-phosphorsäureester (DE-PS 910 652, EP-OS 12 344).

Die Phosphorsäureester der Formel II

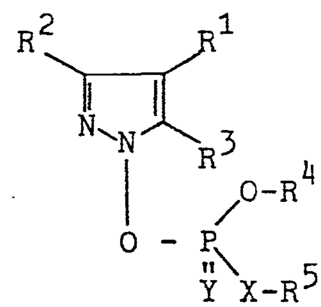

(II),

in der $R^1$, $R^2$ und $R^3$ die obengenannten Bedeutungen haben und $R^4$ und $R^5$ gleiche oder verschiedene $C_1$-$C_6$-Alkylreste, X und Y Sauerstoff oder Schwefel bedeuten, können in an sich bekannter Weise erhalten werden, wenn man (Thio)-Phosphorsäure-(thio)esterhalogenide der Formel IIa

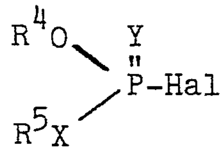

(IIa),

in der $R^4$, $R^5$, X und Y die obengenannten Bedeutungen haben und Hal für Halogen steht,
mit 1-Hydroxipyrazolderivaten der Formel I

BAD ORIGINAL

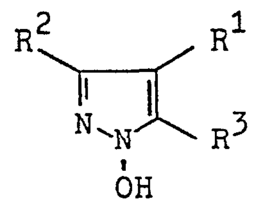
(I),

in der $R^1$, $R^2$ und $R^3$ die obengenannten Bedeutungen haben, in Anwesenheit von Säureakzeptoren oder mit Salzen von 1-Hydroxipyrazolderivaten umsetzt.

Unverzweigte oder verzweigte $C_1$-$C_6$-Alkylreste für $R^4$ und $R^5$ in Formel I sind Methyl, Ethyl, n-Propyl, i-Propyl, i-Butyl, n-Butyl, s-Butyl, n-Pentyl, i-Pentyl, n-Hexyl, i-Hexyl; bevorzugt sind $C_1$-$C_4$-Alkylreste, insbesondere Ethyl.

Die Umsetzung wird bevorzugt in Gegenwart von Lösungsmitteln durchgeführt. Geeignet sind alle inerten Solventien, wie aliphatische und aromatische, gegebenenfalls chlorierte Kohlenwasserstoffe, z.B. Benzin, Toluol, Xylol, Methylenchlorid, Chloroform, Chlorbenzol, Ether, wie Diethyl- oder Dibutylether, Tetrahydrofuran und Dioxan, Ketone, wie Aceton, Methylethyl-, Methylisopropyl- und Methylisobutylketon, und Nitrile, wie Acetonitril oder Propionitril.

Als Säureakzeptoren lassen sich die allgemein üblichen Säurebindemittel einsetzen, wie z.B. Alkalicarbonate bzw. -alkoholate oder aliphatische, aromatische bzw. heterocyclische Amine.

Die Reaktionstemperatur kann zwischen 0 und 100°C variieren; vorzugsweise liegt sie in einem Temperaturbereich zwischen 10 und 60°C.

Die Ausgangsstoffe werden gewöhnlich in äquimolaren Mengen eingesetzt, können aber auch im molaren Unter- oder Überschuß vorliegen.

Die Aufarbeitung des Reaktionsgemisches erfolgt nach üblichen Methoden, z.B. Filtrieren und Abdestillieren des Lösungsmittels, gegebenenfalls nach Extraktion der organischen Phase mit Wasser oder wäßriger $NaHCO_3$- bzw. Sodalösung. Bei Verwendung von wasserlöslichen Solventien gibt man vor der Extraktion ein mit Wasser nicht mischbares Lösungsmittel zu.

Die (Thio)Phosphorsäure-(thio)esterhalogenide sind bekannt und können nach bekannten Verfahren hergestellt werden (DE-OS 26 42 982; J. Org. Chem. 30, 3217 (1965)).

Herstellung von O-Pyrazol-1-yl-O,O-diethylthiophosphat

Zu 55 Teilen 1-Hydroxipyrazol und 50 Teilen Soda in 600 Teilen Acetonitril gibt man unter Rühren bei 25°C 112 Teile Diethoxi-thiophosphorsäurediesterchlorid. Nach der Zugabe rührt man noch 12 Stunden nach. Das Festprodukt wird abgesaugt und zweimal mit 50 Teilen Acetonitril gewaschen. Das Filtrat wird im Rotationsverdampfer bei 20 bis 40°C und 20 mbar eingeengt. Den Rückstand löst man in 300 Gew.-Teilen Methylenchlorid, extrahiert die organische Phase dreimal mit je 50 Gew.-Teilen gesättigter wäßriger $NaHCO_3$-Lösung, trocknet die Methylenchloridlösung mit Magnesiumsulfat und zieht das Lösungsmittel im Rotationsverdampfer bei 20 bis 40°C und 20 mbar ab.

Austrag:
127,5 Gew.-Teile (91 % der Theorie) O-Pyrazol-1-yl-O,O-diethyl-thiophosphat, $H^1$-NMR ($\delta$ in ppm): 1.3 (t, 6H); 4.3 (m, 4H); 6.2 (m, 1H); 7.25 (m, 1H); 7.4 (m, 1H).

Die in der Tabelle angegebenen Substituenten beziehen sich auf jeweils eine Verbindung der Formel II

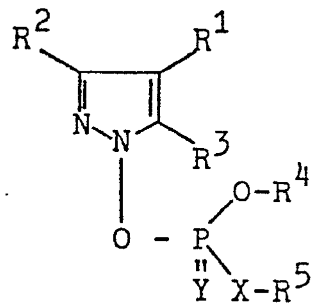

(II)

| Nr. | $R^1$ | $R^2$ | $R^3$ | Y | X | $R^4$ | $R^5$ | $H^1$-NMR ($\delta$ in ppm) |
|---|---|---|---|---|---|---|---|---|
| 1 | Br | H | H | S | S | $C_2H_5$ | i-$C_4H_9$ | 1.0(d, 6H), 1.4(t, 3H), 1.8(m, 1H), 2.8(m, 2H), 4.3(m, 2H), 7.3(s, 1H), 7.5(s, 1H) |
| 2 | H | " | " | " | " | " | " | 1.0(d, 6H), 1.4(t, 3H), 1.9(m, 1H), 2.8(m, 2H), 4.3(m, 2H), 6.2(m, 1H), 7.2(m, 1H), 7.4(m, 1H) |
| 3 | Cl | " | " | " | " | " | " | 1.0(d, 6H), 1.35(t, 3H), 1.9(m, 1H), 2.8(m, 2H), 4.3(m, 2H), 7.2(s, 1H), 7.4(s, 1H) |
| 4 | Br | Br | " | " | " | " | n-$C_3H_7$ | 1.0(t, 3H), 1.4(t, 3H), 1.7(m, 2H), 3.0(m, 2H), 4.35(m, 2H), 7.45(s, 1H) |
| 5 | " | " | Br | " | O | $CH_3$ | $CH_3$ | 3.8(s, 3H), 4.1(s, 3H) |
| 6 | " | H | H | " | " | $C_2H_5$ | $C_2H_5$ | 1.4(t, 6H), 4.3(m, 4H), 7.2(s, 1H), 7.4(s, 1H) |
| 7 | " | Br | " | " | " | " | " | 1.4(t, 6H), 4.3(m, 4H), 7.4(s, 1H) |
| 8 | " | " | " | " | " | $CH_3$ | $CH_3$ | 3.8(s, 3H), 4.1(s, 3H), 7.4(s, 1H) |
| 9 | " | " | Br | " | " | $C_2H_5$ | $C_2H_5$ | 1.3(m, 6H), 4.3(m, 4H) |
| 10 | " | " | " | " | S | " | n-$C_3H_7$ | 0.7-1.7(m, 8H), 2.7(m, 2H), 4.35(m, 2H) |
| 11 | Cl | H | H | " | O | $CH_3$ | $CH_3$ | 3.8(s, 3H), 4.0(s, 3H), 7.25(s, 1H), 7.4(s, 1H) |
| 12 | Br | " | " | " | " | " | " | 3.8(s, 3H), 4.0(s, 3H), 7.2(s, 1H), 7.4(s, 1H) |
| 13 | " | " | " | " | S | $C_2H_5$ | n-$C_3H_7$ | 1.0(t, 3H), 1.4(t, 3H), 1.7(m, 2H), 3.0(m, 2H), 4.3(m, 2H), 7.2(s, 1H), 7.45(s, 1H) |
| 14 | Cl | " | " | " | O | " | $C_2H_5$ | 1.4(t, 6H), 4.35(m, 4H), 7.25(s, 1H), 7.45(s, 1H) |

| Nr. | $R^1$ | $R^2$ | $R^3$ | Y | X | $R^4$ | $R^5$ | $H^1$-NMR (δ in ppm) |
|---|---|---|---|---|---|---|---|---|
| 15 | H | H | H | O | O | $C_2H_5$ | $C_2H_5$ | 1.35(t, 6H), 4.3(m, 4H), 6.2(m, 1H), 7.2(m, 1H), 7.4(m, 1H) |
| 16 | " | " | " | S | " | " | " | 1.4(t, 6H), 4.3(m, 4H), 6.2(m, 1H), 7.25(m, 1H), 7.4(m, 1H) |
| 17 | " | " | " | " | " | $CH_3$ | $CH_3$ | 3.8(s, 3H), 4.0(s, 3H), 6.2(m, 1H), 7.25(m, 1H), 7.4(m, 1H) |
| 18 | " | " | " | " | S | $C_2H_5$ | n-$C_3H_7$ | 1.0(t, 3H), 1.4(t, 3H), 1.75(m, 2H), 3.0(m, 2H), 4.35(m, 2H), 6.2(m, 1H), 7.25(m, 1H), 7.4(m, 1H) |
| 19 | Cl | " | " | " | " | " | " | 1.0(t, 3H), 1.4(t, 3H), 1.7(m, 2H), 3.0(m, 2H), 4.35(m, 2H), 7.2(s, 1H), 7.5(s, 1H) |
| 20 | I | " | " | " | O | " | $C_2H_5$ | 1.4(t, 6H), 4.3(m, 4H), 7.3(s, 1H), 7.5(s, 1H) |
| 21 | " | " | " | " | " | $CH_3$ | $CH_3$ | 3.8(s, 3H), 4.05(s, 3H), 7.35(s, 1H), 7.45(s, 1H) |
| 22 | Cl | Br | Br | " | " | $C_2H_5$ | $C_2H_5$ | 1.4(m, 6H), 4.4(m, 4H) |
| 23 | " | " | " | " | " | $CH_3$- | $CH_3$- | 3.85(bs, 3H), 4.15(bs, 3H) |
| 24 | Br | H | H | " | S | $C_2H_5$ | s-$C_4H_9$ | 0.8-2.0(m, 11H), 3.6(m, 1H), 4.3(m, 2H), 7.3(s, 1H), 7.5(s, 1H) |
| 25 | H | " | " | " | " | " | " | 0.8-2.1(m, 11H), 3.6(m, 1H), 4.4(m, 2H), 6.3(m, 1H), 7.3(s, 1H), 7.5(s, 1H) |
| 26 | Cl | Cl | Cl | " | O | " | $C_2H_5$ | 1.35(m, 6H), 4.35(m, 4H) |
| 27 | I | I | I | " | " | " | " | 1.3(m, 6H), 4.3(m, 4H) |

| Nr. | $R^1$ | $R^2$ | $R^3$ | Y | X | $R^4$ | $R^5$ | $H^1$-NMR ($\delta$ in ppm) |
|---|---|---|---|---|---|---|---|---|
| 28 | H | H | H | O | O | $C_2H_5$ | n-$C_6H_{13}$ | |
| 29 | Cl | H | H | S | S | $C_2H_5$ | i-$C_5H_{11}$ | |
| 30 | I | H | H | S | O | $C_2H_5$ | n-$C_5H_{11}$ | |
| 31 | H | H | H | O | O | n-$C_6H_{13}$ | $C_2H_5$ | |
| 32 | Cl | Cl | H | S | O | $C_2H_5$ | i-$C_6H_{13}$ | |
| 33 | Br | H | H | S | O | $C_2H_5$ | $C_2H_5$ | |
| 34 | H | H | H | O | S | $C_2H_5$ | i-$C_4H_9$ | |

Bekämpfbare Insekten und Spinnentiere, Formulierungshinweise und Angaben über empfehlenswerte Mischungspartner können den Angaben in der DE-OS 30 39 080 bzw. der EP-Veröffentlichung 0050219 entnommen werden, die sich mit strukturell verwandten Wirkstoffen beschäftigt.

Wie die folgenden Testergebnisse zeigen, eignen sich diese 1-Pyrazolylphosphorsäureester zur Bekämpfung von Schädlingen aus der Klasse der Insekten und Spinnentiere. Die Numerierung der Wirkstoffe entspricht der der tabellarischen Auflistung.

Beispiel 1

Fraß- und Kontaktwirkung auf Raupen der Kohlschabe (Plutella maculipennis)

Blätter von jungen Kohlpflanzen werden 3 Sekunden lang in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt. Nach 48 Stunden beurteilt man die Wirkung.

In diesem Test zeigen die Wirkstoffe Nr. 1, 2, 3, 6, 14, 16 eine überlegene Wirkung gegenüber dem Vergleichsmittel.

Beispiel 2

Kontaktwirkung auf Schaben (Blatta orientalis)

Der Boden eines 1 l-Einmachglases wird mit der acetonischen Lösung des Wirkstoffs behandelt. Nach dem Verdunsten des Lösungsmittels setzt man je Glas 5 adulte Schaben. Die Mortalitätsrate wird nach 48 Stunden bestimmt.

In diesem Test zeigen die Wirkstoffe Nr. 2, 6, 7, 11, 12, 14, 15, 16, 18 eine gute Wirkung.

Beispiel 3

Kontaktwirkung auf Zecken (Ornithodorus moubata)

Geprüft wird an jungen Zecken, die erst einmal Blut aufgenommen haben. Dazu taucht man je 5 Tiere, die sich in einem Papierbeutel befinden, für 5 Sekunden in die wäßrige Wirkstoffaufbereitung. Die Beutel werden frei aufgehängt. Die Versuchstemperatur beträgt 25 bis 26°C. Nach 48 Stunden ermittelt man die Mortalitätsrate.

In diesem Test zeigen die Wirkstoffe Nr. 2, 6, 7, 14, 15, 16, 17, 18 eine bessere Wirkung als das Vergleichsmittel.

Beispiel 4

Kontaktwirkung auf Baumwollwanzen (Dysdercus intermedius)

Petrischalen von 10 cm Durchmesser werden mit 1 ml acetonischer Wirkstofflösung ausgekleidet.

Nach dem Verdunsten des Lösungsmittels besetzt man die Schalen mit je 20 Larven des vorletzten Stadiums und registriert die Wirkung nach 24 Stunden.

In diesem Test zeigen die Wirkstoffe Nr. 1, 2, 3, 6, 7, 8, 9, 11, 12, 13, 14, 15, 16, 18 und 20 gute Wirkung.

Beispiel 5

Systemische Wirkung auf Raupen (Prodenia litura)

200 ml Quarzsand werden in 250 ml-Kunststoffbecher in Paletten zu 8 Gefäßen gegeben. Man belegt jeden Becher mit 5 Maiskörnern (ca. 1 cm unter die Oberfläche). Darauf befeuchtet man mit je 50 ml Wasser und deckt mit einer passenden transparenten Kunststoffhaube ab. Nach 8 Tagen werden die Paletten abgedeckt, nach 10 Tagen erfolgt die Behandlung. Dabei gießt man 40 ml der wäßrigen Wirkstoffaufbereitung an die Pflanzen und deckt nach einem weiteren Tag mit 50 ml trockenem Quarzsand ab. Hierdurch soll ein Kontakt der Versuchstiere mit der behandelten Oberfläche vermieden werden.

Auf jeden Becher stellt man einen Plastik-Zylinder (Durchmesser 7 cm), belegt mit je 5 Raupen im 3. Larvenstadium

und deckt den Zylinder mit einem Drahtgazedeckel ab. Nach 4 Tagen beurteilt man Fraß und Mortalität in den Gefäßen.

In diesem Test zeigen die Wirkstoffe Nr. 2, 3, 6, 9, 13, 14, 16, eine bessere Wirkung als das Vergleichsmittel.

Beispiel 6

Kontaktwirkung auf Blattläuse (Aphis fabae); Spritzversuch

Getopfte Bohnenpflanzen (Vicia faba) mit starken Blattlaufkolonien werden in einer Spritzkammer mit wäßrigen Wirkstoffaufbereitungen tropfnaß gespritzt. Die Auswertung erfolgt nach 24 Stunden.

In diesem Test zeigen die Wirkstoffe Nr. 1, 2, 3, 6, 7, 12, 13, 14, 15, 16, 17, 18 und 20 eine gute Wirkung.

Beispiel 7

Kontaktwirkung auf Stubenfliegen (Musca domestica); Dauerkontakt

Beide Teile einer Petrischale von 10 cm Durchmesser werden mit insgesamt 2 ml der acetonischen Wirkstofflösung ausgekleidet. Nach dem Verdunsten des Lösungsmittels (ca. 30 Minuten) bringt man je 20 4-Tage alte Fliegen in die Schalen. Die Mortalitätsrate wird nach 4 Stunden festgestellt.

In diesem Test wird mit den Wirkstoffen Nr. 1, 2, 6, 7, 13, 14, 15, 16, 18 und 20 eine höhere Mortalitätsrate erzielt als mit dem Vergleichsmittel.

Beispiel 8

Kontaktwirkung auf Stubenfliegen (Musca domestica); Applikationstest

4-Tage alte Imagines erhalten in leichter $CO_2$-Narkose 1 µl der acetonischen Lösung des Wirkstoffes auf das ventrale Abdomen appliziert. Hierzu wird eine Mikrometerspitze verwendet.

Je 20 Versuchstiere mit gleicher Behandlung bringt man dann in einen Folienbeutel von ca. 500 ml Inhalt. Nach 4 Stunden zählt man die Tiere in Rückenlage aus und ermittelt graphisch die LD 50.

Die LD 50 der Wirkstoffe Nr. 6, 7, 15, 16 ist niedriger als die des Vergleichsmittel.

## Patentansprüche

1. Halogenierte 1-Hydroxipyrazole der Formel

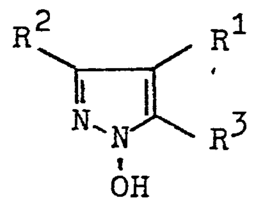

(I),

in der
$R^1$, $R^2$ und $R^3$ unabhängig voneinander Wasserstoff, Chlor, Brom oder Iod bedeuten, mit der Maßgabe, daß $R^1$, $R^2$ und $R^3$ nicht gleichzeitig für Wasserstoff stehen.

2. 1-Pyrazolyl-phosphorsäureester der Formel

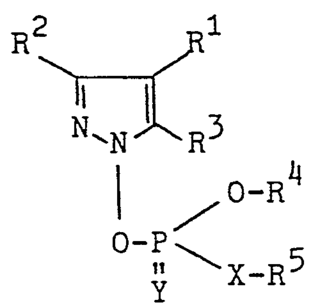

(II),

in der

| | |
|---|---|
| $R^1$, $R^2$, $R^3$ | gleich oder verschieden sind und Wasserstoff, Chlor, Brom oder Iod, |
| $R^4$, $R^5$ | gleiche oder verschiedene $C_1$-$C_6$-Alkylreste bedeuten und |
| X, Y | für Sauerstoff oder Schwefel stehen. |

3. 1-Pyrazolyl-phosphorsäureester der Formel II gemäß Anspruch 2, dadurch gekennzeichnet, daß $R^1$ Wasserstoff, Chlor oder Brom, $R^2$ Wasserstoff, $R^3$ Wasserstoff, $R^4$ Ethyl, $R^5$ Ethyl, X Sauerstoff und Y Sauerstoff oder Schwefel bedeuten.

4. Verfahren zur Herstellung von 1-Pyrazolyl-phosphorsäureestern der Formel II gemäß Anspruch 2, dadurch gekennzeichnet, daß man (Thio)Phosphorsäure-(thio)-esterhalogenide der Formel

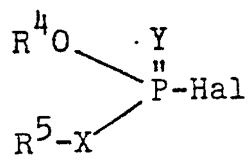

(IIa),

in der
$R^4$, $R^5$, X und Y die im Anspruch 2 genannten Bedeutungen haben und Hal für Halogen steht,
mit 1-Hydroxipyrazol bzw. -derivaten der Formel

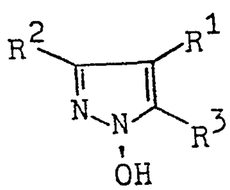

(I),

in der
$R^1$, $R^2$ und $R^3$ die im Anspruch 2 genannten Bedeutungen haben,
in Anwesenheit von Säureakzeptoren oder mit Salzen von 1-Hydroxipyrazol bzw. -derivaten umsetzt.

5. Schädlingsbekämpfungsmittel, enthaltend einen 1-Pyrazolyl-phosphorsäureester der Formel II gemäß Anspruch 2.

6. Schädlingsbekämpfungsmittel, enthaltend inerte Zusatzstoffe und einen 1-Pyrazolyl-phosphorsäureester der Formel II gemäß Anspruch 2.

7. Verwendung von 1-Pyrazolyl-phosphorsäureestern der Formel II gemäß Anspruch 2 zur Bekämpfung von Schädlingen.

8. Verfahren zur Bekämpfung von Schädlingen, dadurch gekennzeichnet, daß man eine wirksame Menge eines 1-Pyrazolylphosphorsäureesters der Formel II gemäß Anspruch 2 auf die Schädlinge und/oder deren Lebensraum einwirken läßt.

Europäisches Patentamt

# EUROPÄISCHER RECHERCHENBERICHT

0087615
Nummer der Anmeldung

EP 83 10 1128

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | US-A-2 888 462 (W.H. CANNON)<br>--- | 1,2,5, 6,7,8 | C 07 D 231/16<br>C 07 F 9/65<br>A 01 N 57/16 //<br>C 07 D 261/08 |
| A | JOURNAL OF ORGANIC CHEMISTRY, Band 45, Nr. 1, 1980, Seiten 76-80, American Chemical Society, USA<br>J.F. HANSEN et al.: "Halogenation of N-oxygenated pyrazoles. Preparation of N-oxygenated 4-halopyrazole and 4,4-dihalo-4H-pyrazole derivatives" * Seiten 76-80 *<br>----- | 1 | |
| | | | RECHERCHIERTE SACHGEBIETE (Int. Cl. 3) |
| | | | C 07 D 231/00<br>C 07 F 9/00 |

Der vorliegende Recherchenbericht wurde für alle Patentansprüche erstellt.

| Recherchenort | Abschlußdatum der Recherche | Prüfer |
|---|---|---|
| DEN HAAG | 23-06-1983 | CREMERS K. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument